# EUROPEAN PATENT APPLICATION

(11) **EP 4 122 446 A1**
(43) Date of publication of application: **25.01.2023**
(21) Application number: 21772135.6
(22) Date of filing: 12.03.2021
(51) Int. Cl.: A61K 9/06, A61K 47/69, A61K 39/395, C07K 16/28, A61P 35/00

(54) **DEFORMABLE HYDROGEL PARTICLES AND PHARMACEUTICAL COMPOSITION FOR CANCER TREATMENT COMPRISING SAME**

(30) Priority: 16.03.2020 KR 20200032035; 10.12.2020 KR 20200172631
(71) Applicant: Scholar Foxtrot Co., Ltd., Seoul 02796 (KR); Korea University Research and Business Foundation, Seoul 02841 (KR)
(72) Inventor: KIM, Jongseong, Goyang-si Gyeonggi-do 10321 (KR); CHAE, Yunjin, Seoul 02767 (KR)
(74) Representative: ZBM Patents - Zea, Barlocci & Markvardsen
(86) International application number: PCT/KR2021/003095
(87) International publication number: WO 2021/187813

(57) **Abstract**

The objective of the present invention is to provide hydrogel particles and a pharmaceutical composition for cancer treatment comprising the same, the hydrogel particles being deformable and having bound to the surfaces thereof a protein capable of binding to cell surface components of cancer cells and/or T cells.

## Description

### Technical Field

### Statement of government-supported research and development

The present invention was made with government support under research project number HI14C 3477 granted by the Ministry of Health and Welfare.

The following description relates to deformable hydrogel particles capable of preventing the immune evasion mechanism of cancer cells, and a pharmaceutical composition for treating cancer comprising the same.

### Background Art

The human body's immune system consists of various organs and special cells and substances that have an immune effect. Immune cells and immune substances are responsible for inhibiting inflammation caused by antigens that stimulate immune responses to foreign substances, bacteria, or the like that are not derived from the human body and suppressing cancer cells. T-cells and B-cells are representative cells responsible for immunity. Depending on the type, T-cells either directly attack antigens or help B-cells to function. B-cells secret antibodies that can attack the antigen to eliminate the antigen.

Cancer cells may use immune checkpoints to evade the surveillance of the immune system. Immune checkpoint protein is a protein that activates or inactivates immune cells in our body and is a medium in which the immune system distinguishes between cancer cells and normal cells. Representative checkpoint proteins are programmed death 1 (PD-1) and cytotoxic T-lymphocyte-associated antigen 4 (CTLA-4) on the T cell surface. When they meet proteins on the surface of normal cells (e.g., PD-L1 and B7), they inactivate T cells to prevent an attack on normal cells. Cancer cells evade attacks by the immune system by expressing checkpoint proteins such as PD-L1 and B7.

Immuno-oncology agents inhibit cancer cells from evading the surveillance of the immune system or enhance the action of immune cells so that immune cells can attack cancer cells more effectively. An anti-PD-1 antibody, anti-PD-L1 antibody, anti-CTLA-4 antibody, etc., have been approved by FDA and are being used in clinical practice. However, such antibody-based immuno-oncology agents require a large amount to be administered to regulate immune checkpoint molecular functions, which not only causes toxicity and side effects but also accompanies high treatment costs.

As another immunotherapy-based cancer treatment method, a treatment that injects a gene (chimeric antigen receptor, CAR) designed to recognize cancer cells and signal the immune activation of T cells into T-cells isolated from a patient's blood has received FDA approval. The completed CAR-T cells are proliferated into millions and then administered to the patient again. This process takes a lot of time, and the risk of infection and the cost are also considerably high.

In order to take advantage of CAR-T-based cancer immunotherapy, but relatively inexpensive single antibody-based immuno-oncology agent compared to CAR-T, bispecific- or trispecific-antibody technologies are being developed. The bispecific antibody and the trispecific antibody exhibit superior cancer cell killing activity through the function of two or more antibodies compared to a monospecific antibody. However, to implement this, recombinant protein technology is used, which takes a lot of time and cost.

Meanwhile, studies for using hydrogel as a drug delivery means are being actively conducted. A hydrogel is a three-dimensional structure composed of a network of hydrophilic polymers, and more than 90% of the components are water. Hydrogels are attracting attention as a drug delivery means in the pharmaceutical field because of their properties similar to biological tissues, such as high-water content, porous structure, relatively soft physical properties, and biocompatibility.

Hydrogels may exhibit various properties depending on the type of polymer used as the main chain, and the cross-linking method. For example, a stimuli-responsive polymer may be used to form a hydrogel that responds to a specific stimulus. Polymers having many ionizing functional groups may be used to form hydrogels whose physical properties can be changed by a change in pH. Polymers that undergo structural transformation by specific stimuli such as temperature or light may be used to form hydrogels whose physicochemical behavior changes in response to stimuli. As with the type of polymer used, the cross-linking method affects the properties of the hydrogel. Even if the same polymer is used as the main chain, hydrogels with completely different properties can be obtained if the cross-linking methods are different. The method of cross-linking hydrogels is largely divided into physical and chemical cross-linking methods. Physical cross-linking methods include ionic interactions, hydrophobic interactions, hydrogen bonds, and reversible cross-linking methods due to structurally molecular entanglement. These cross-linking methods may easily induce the formation of a three-dimensional network internal structure without the need for a separate chemical additive or complicated process for cross-linking. On the other hand, the chemical cross-linking methods are generally an irreversible method by covalent bonding and form a stable network compared to physical cross-linking methods. As with stimuli-sensitive polymers, in hydrogels containing a crosslinker that structurally deform or decompose by stimulation such as pH change, temperature, light, or ultrasound, the physical properties of the gel can be controlled by external stimulation (Jisoo Shin et al., "Functional Hydrogel for the Application of Drug Delivery and Tissue Engineering," KIC News, Vol. 18, No. 6, (2015): pages 2-3).

Korean Patent No. 10-1754774 discloses a biochip using a hydrogel whose physical properties are changed by a specific stimulus. A binding-mediated substrate is formed on the surface of the hydrogel, and when the binding-mediated substrate binds to a target protein, de-swelling occurs in the hydrogel, which results in physical properties of the hydrogel being changed (e.g., refractive index, volume, etc.). The changed physical properties are transmitted to the analysis equipment as a corresponding displacement signal. The displacement signal is analyzed to measure the number of multiple bonds between the target protein and the binding-mediated substrate, thereby functioning as a biochip. However, this document does not disclose the use of the described hydrogel as a drug delivery agent or an anticancer therapeutic agent using the same.

### [Prior art document]

### Patent document

(Patent Document 1) Korea Patent No. 10-1754774

### Non-patent document

(Non-patent document 1) Jisoo Shin et al., "Functional Hydrogel for the Application of Drug Delivery and Tissue Engineering," KIC News, Vol. 18, No. 6, (2015): pages 2-3.

### Disclosure of the Invention

### Technical Goals

Under this technical background, the present inventors have developed hydrogel-based, deformable immuno-oncology agent particles that act like artificial T-cells, which can prevent the immune system evasion mechanism of cancer cells by binding to cancer cells and/or T-cells to block the interaction between them.

Accordingly, an object of the present invention is to provide a hydrogel particle which is deformable and has a protein capable of binding to cell surface components of cancer cells and/or T-cells, wherein the protein is bound to the surface of the hydrogel particle, and an immuno-oncology agent or pharmaceutical composition for cancer treatment comprising the same.

However, the technical goal to be achieved by the present invention is not limited to the technical goals mentioned above, and other technical goals not mentioned are clearly understood by those of ordinary skill in the art from the following description.

### Technical Solutions

An aspect of the present invention relates to a hydrogel particle, characterized in that a protein capable of binding to a cell surface component of cancer cells and/or T-cells is bound to the surface of the hydrogel particle, and the hydrogel particle is deformable.

According to one example embodiment, the cell surface component includes at least one selected from the group consisting of CD2, CD3, CD19, CD24, CD27, CD28, CD31, CD34, CD45, CD46, CD80, CD86, CD133, CD134, CD135, CD137, CD160, CD335, CD337, CD40L, ICOS, GITR, HVEM, Galectin 9, TIM-1, LFA-1, PD-L1, PD-L2, B7-H3, B7-H4, ILT3, ILT4, PD-1, CTLA-4, BTLA, MHC-I, MHC-II, TGF-β-receptor, latent TGF-β-binding protein (LTBP), delta-like ligand (for example, DLL-Fc, DLL-1, or DLL-4), WNT3, stem cell factor, and thrombopoietin.

According to one aspect, the cell surface component of the cancer cell can be PD-L1 protein, and the cell surface component of the T-cell can be selected from the group consisting of PD-1 protein, CTLA-4 protein, and CD137 protein.

According to one aspect, the protein bound to the surface of the hydrogel can be an antibody, a recombinant protein, or a combination thereof.

According to one aspect, the antibody can be an anti-PD-1 antibody, an anti-PD-L1 antibody, an anti-CD137 antibody, an anti-CTLA-4 antibody, or a combination thereof.

According to one aspect, the recombinant protein can comprise at least one selected from the group consisting of a protein, an aptamer, or a combination thereof, and in which the protein is capable of targeting and binding at least one selected from the group consisting of PD-L1 protein, PD-1 protein, CTLA-4 protein, and CD137 protein.

According to one aspect, the hydrogel is a nanoparticle. Specifically, the diameter of the hydrogel in deionized water may range from about 50 nm to about 3,000 nm, for example, about 100 nm to about 2,500 nm, or about 300 nm to about 2,000 nm, preferably 440 nm or more, more preferably about 540 nm or more, even more preferably 700 nm or more, and for example, about 700 nm to about 1,300 nm.

According to an aspect, the hydrogel can comprise a synthetic copolymer consisting of the main monomer and a comonomer. For example, the main monomer can be selected from the group consisting of N-isopropylacrylamide, N-acryloylglycinamide, hydroxypropylcellulose, vinylcaprolactame, N-vinyl pyrrolidone, 2-hydroxyethyl methacrylate, ethylene glycol; amino acids such as aspartic acid, glutamic acid, and L-lysine; caprolactone, and vinyl methyl ether, and in which the comonomer is selected from the group consisting of allylamine (AA), dimethylaminoethyl methacrylate (DMAEMA), dimethylaminoethyl acrylate (DMAEA), acrylic acid (AAc), ethylene glycol (EG), and methacrylic acid (MAAc).

According to one aspect, the hydrogel can comprise a synthetic homopolymer composed of a single monomer. Exemplary homopolymers can comprise poly(ethylene glycol) (PEG), poly(2-methyl-2-oxazoline) (PMOXA), polyethylene oxide) (PEO), poly(vinyl alcohol) (PVA) and poly(acrylamide) (PAAm), poly(n-butylacrylate), poly-(α-ester), poly(glycolic acid) (PGA), polyaspartate, polyglutamate, polylactide, poly(N-isopropylacrylamide) (pNIPAAM), poly(caprolactone), polyvinylmethyl ether, and the like.

According to one aspect, the hydrogel can further comprise a cross-linking agent. The cross-linking agent can be selected from the group consisting of N,N'-methylene-bis-diacrylamide (MBA), polyethylene glycol (PEG) PEG dihydroxyl, PEG diamine, PEG dioxyamine, PEG dichloride, PEG dibromide, PEG diazide, PEG dithiol, PEG dialdehyde, PEG diepoxide, PEG diacrylate, PEG dimethacrylate, PEG diacetic acid, PEG disuccinic acid, PEG discuccinimidyl carboxy methyl ester, poly(ε-caprolactone)diacrylate, poly(ε-caprolactone)dimethacrylate, polylactide diacrylate, polylactide dimethacrylate, poly(lactide-co-glycolide)diacrylate, poly(lactide-co-glycolide)dimethacrylate, poly(ε-caprolactone-b-ethylene glycol-b-ε-caprolactone)diacrylate, poly(ε-caprolactone-b-ethylene glycol-b-ε-caprolactone)dimethacrylate, poly(lactide-b-ethylene glycol-b-lactide)diacrylate, poly(lactide-b-ethylene glycol-b-lactide)dimethacrylate, poly[(lactide-co-glycolide)-b-ethylene glycol-b-(lactide-co-glycolide)] diacrylate, poly[(lactide-co-glycolide)-b-ethylene glycol-b-(lactide-co-glycolide)] dimethacrylate, poly(s-caprolactone-co-lactide)-diacrylate, poly(ε-caprolactone-co-lactide)-dimethacrylate, poly(ε-caprolactone-co-glycolide)-diacrylate, poly(ε-caprolactone-co-glycolide)-dimethacrylate, poly[(caprolactone-co-lactide)-b-ethylene glycol-b-(caprolactone-co-lactide)]diacrylate, poly[(caprolactone-co-lactide)-b-ethylene glycol-b-(caprolactone-co-lactide)]dimethacrylate, poly[(caprolactone-co-glycolide)-b-ethylene glycol-b-(caprolactone-co-glycolide)]diacrylate, poly[(caprolactone-co-glycolide)-b-ethylene glycol-b-(caprolactone-co-glycolide)]dimethacrylate and a combination thereof.

According to one aspect, the hydrogel comprises a synthetic copolymer obtained by copolymerizing a main monomer, a comonomer, and a cross-linking agent, for example, can comprise 50 to 97.9% by weight of the main monomer, 2 to 40% by weight of the comonomer, and 0.1 to 10% by weight of a cross-linking agent.

According to one aspect, the hydrogel can comprise at least one selected from the group consisting of poly(N-isoprophylacrylamide-co-allylamine) (poly(NIPAM-co-AA), poly(N-isopropylacrylamide-co-2-(dimethylamino)ethyl methacrylate) (poly(NIPAM-co-DMAEMA)), poly(N-isopropylacrylamide-co-2-(dimethylamino)ethyl acrylate) (poly(NIPAM-co-DMAEA)), poly(N-isopropylacrylamide-co-acrylic acid) (poly(NIPAM-co-AAc)), poly(N-isopropylacrylamide-co-polyethylene glycol-acrylic acid) (poly(NIPAM-co-PEG-AAc)), and poly(N-isopropylacrylamide-co-methacrylic acid) (poly(NIPAM-co-MAAc)).

According to one aspect, the hydrogel can comprise a natural polymer. Exemplary natural polymers can comprise alginate, agarose, carrageenan, chitosan, dextran, carboxymethylcellulose, heparin, hyaluronic acid, polyamino acids, collagen, gelatin, fibrin, fibrous protein-based biopolymers (e.g., silk, keratin, elastin, and resilin), and combinations thereof.

According to one aspect, the protein bound to the hydrogel surface can be bound by at least one linkage selected from the group consisting of carbodiimide cross-linking, Schiff base cross-linking, Azlactone cross-linking, carbonyl diimidazole (CDI) cross-linking, iodoacetyl cross-linking, hydrazide cross-linking, Mannich cross-linking, and maleimide cross-linking, to the surface of the hydrogel.

According to one aspect, the protein bound to the hydrogel surface can be bound to at least one selected from the group consisting of another protein, an aptamer, or a combination thereof that can target and bind one or more cell surface components by using standard binding methods such as protein A : Fc interaction, protein G : Fc interaction, protein A/G : Fc interaction, or maleimide/thiol, and EDC/NHS coupling.

Another embodiment of the present invention relates to a method for producing a hydrogel particle being deformable and having a protein capable of binding to a cell surface component of cancer cells and/or T-cells, bound to the surface of the hydrogel particle, in which the method can comprise steps of:
(i) preparing a hydrogel particle,
(ii) modifying the surface of the hydrogel particle so that the protein can bind to the surface of the hydrogel particle, and
(iii) adding a protein to the surface-modified hydrogel particle, thereby binding the protein to the surface of the hydrogel particle.

An additional embodiment of the present invention relates to an immuno-oncology agent or pharmaceutical composition for treating cancer, comprising a therapeutically effective amount of a deformable hydrogel particle, and a method for treating cancer, the method comprising a step of administering the immuno-oncology agent or pharmaceutical composition to a patient.

### Advantageous Effect

The deformable particle according to the present disclosure is based on a soft hydrogel, to the surface of which a protein capable of binding to cell surface components of cancer cells and/or T-cells, in particular an immune checkpoint protein, is bound. When the protein bound to the hydrogel surface binds to the cell surface components of cancer cells and/or T-cells, the deformable particles according to the present disclosure attach to the cancer cells and/or T-cells to block the interaction between the two cells, thereby preventing the immune evasion mechanism of cancer cells and promoting cancer cell death.

In particular, due to the mechanical softness of the soft hydrogel due to the components constituting the hydrogel in the present disclosure, when the hydrogel comes into contact with the cells, the physical shape deformation of the hydrogel occurs, covering the cell surface and increasing the contact area. Thus, by way of area inhibition, it is possible to greatly reduce the binding possibility of the immune checkpoint proteins of cancer cells and T-cells with a smaller amount of antibodies compared to the existing immuno-oncology agents.

In addition, since two or more types of antibodies can be bound to the deformable particles of the present invention as well as a single antibody, the effect of multi-specific antibodies can be achieved through a simpler manufacturing process compared to recombinant protein technology.

It should be understood that the effects of the present invention are not limited to the above-described effects and include all effects that can be inferred from the technical elements of the invention described in the detailed description or claims of the present invention.

### Brief Description of Drawings

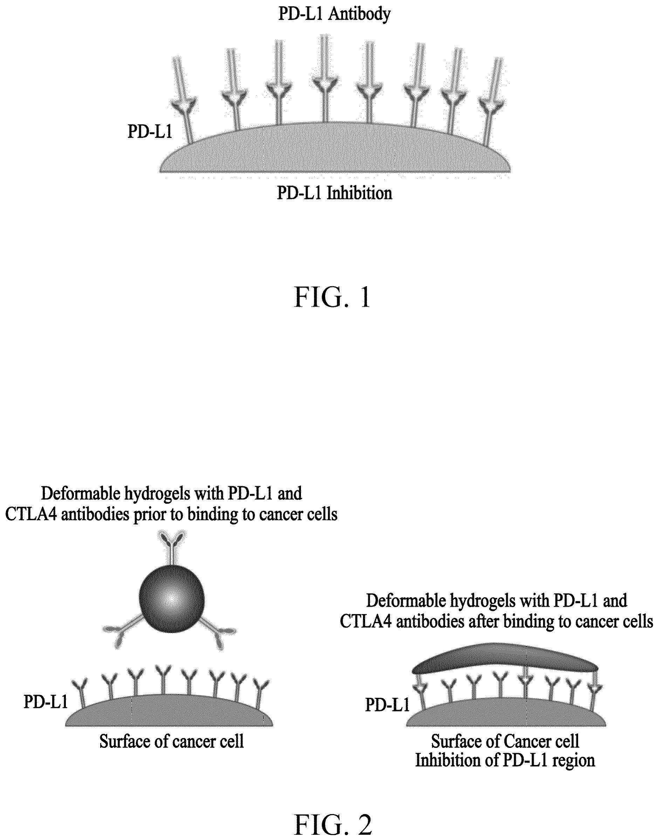

FIG. 1 is a schematic view illustrating the mechanism of the conventional immuno-oncology agent.
FIG. 2 is a schematic view showing that the deformable particles according to the present disclosure bind to the cancer cell surface to act through area inhibition covering the cancer cell surface protein, thereby blocking the interaction between the cancer cell surface protein and the T-cell surface protein to preventing immune evasion of cancer cells.

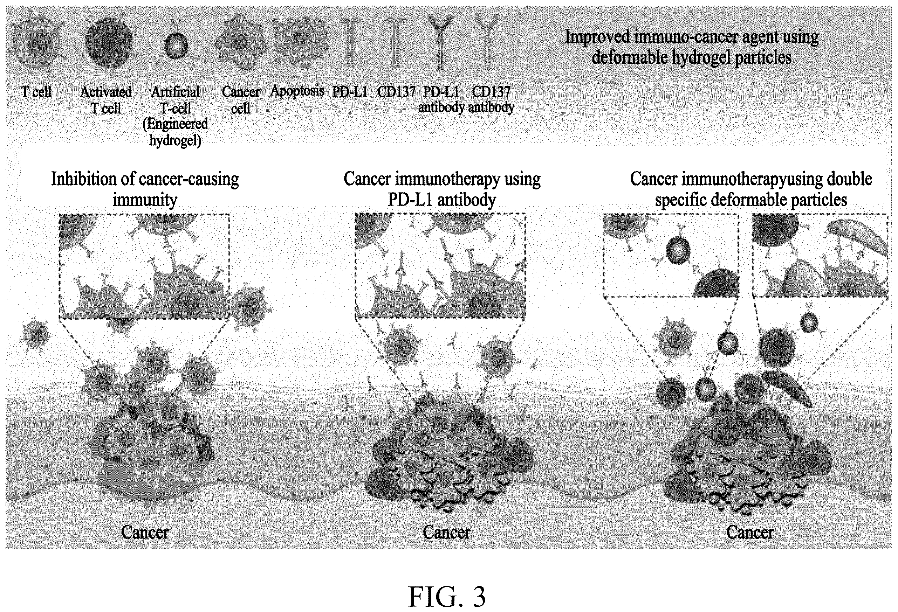

FIG. 3 is a view showing a comparison of the anticancer mechanism using the conventional immuno-oncology agent and the deformable particle according to the present disclosure.

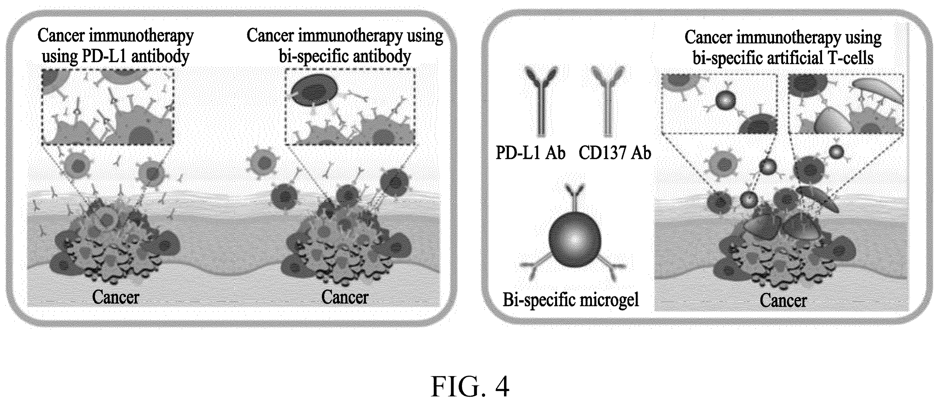

FIG. 4 is a view showing a comparison between the conventional anticancer mechanism using a mono- or bi-specific antibody (left view) and the anticancer mechanism using the deformable particle according to the present disclosure (right view).

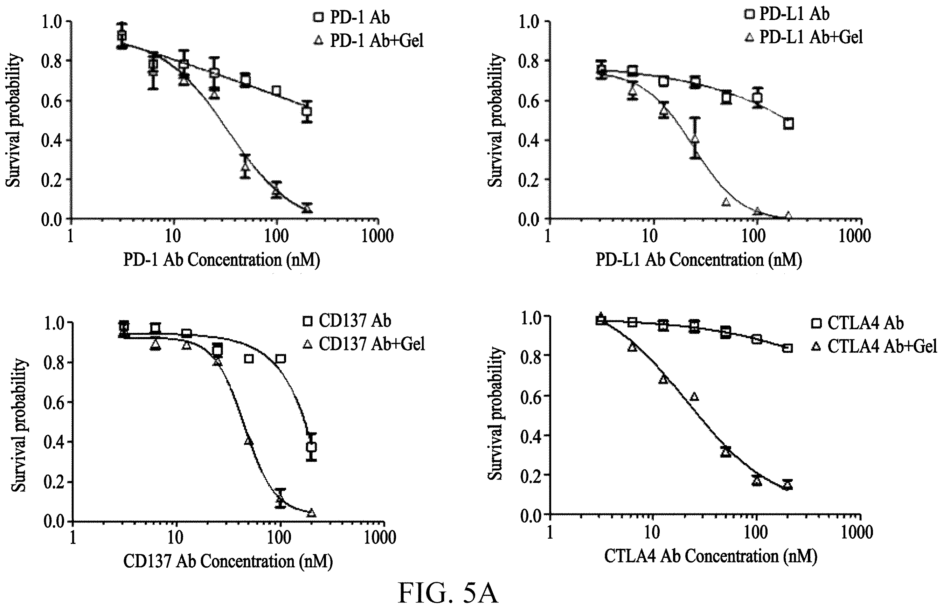

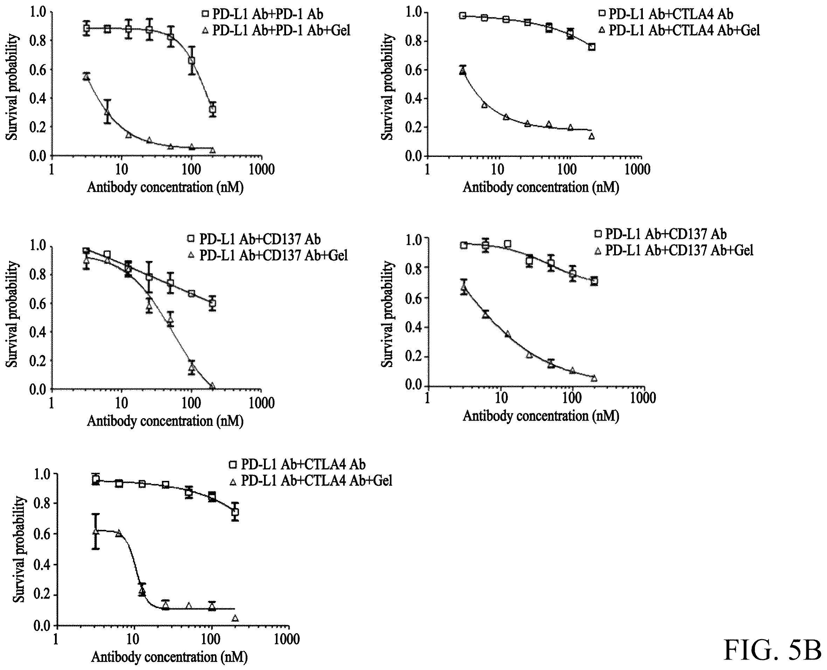

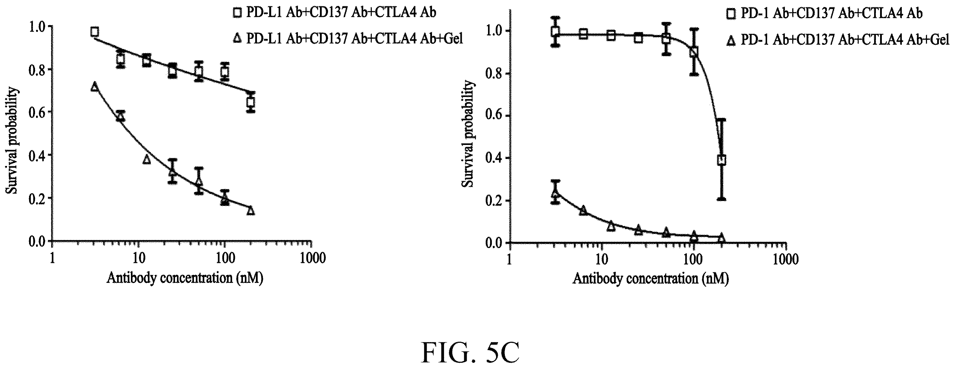

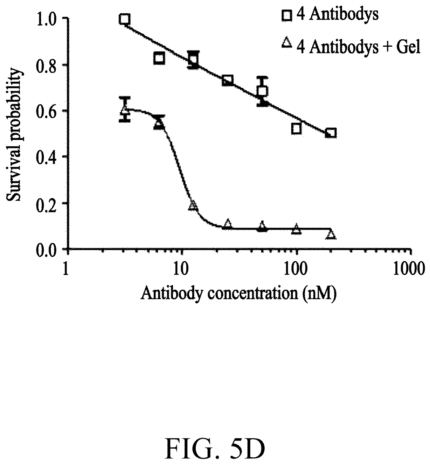

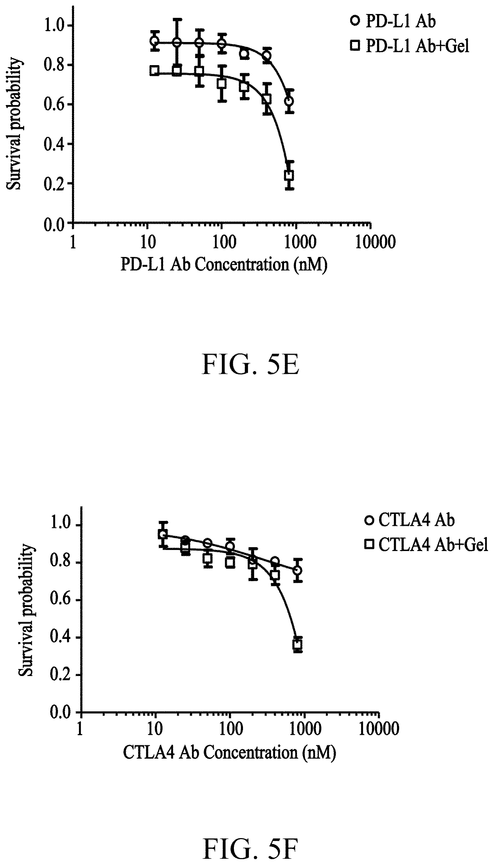

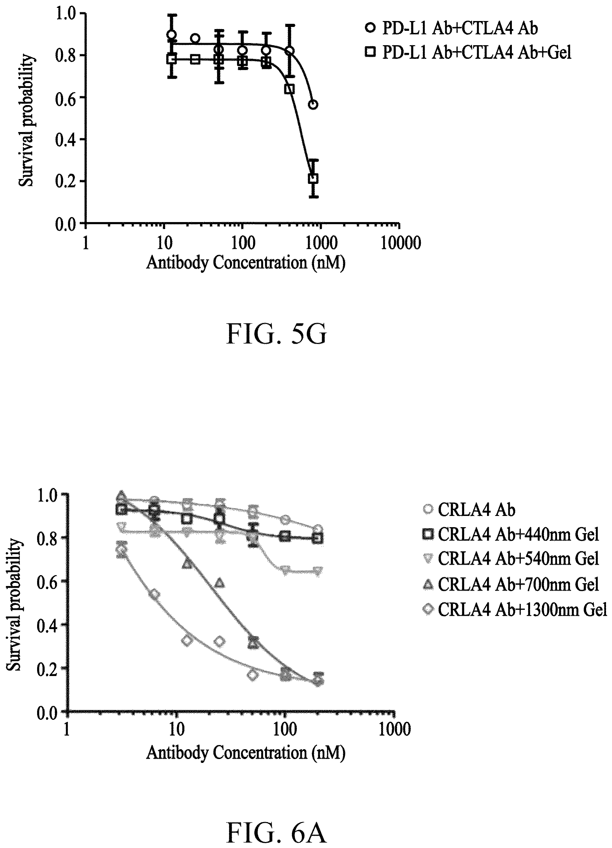

FIG. 5 is a view confirming the survival probability of cancer cells according to the concentration of the treated antibody when the cancer cells are treated with the antibody alone or treated with the antibody-bound deformable particles according to the present disclosure and then cultured with immune cells: FIGS. 5A to 5D relate to the survival probability of breast cancer cells;, FIG. 5A is a view showing a comparison of the cases in which the cells were treated with anti-PD-L1 antibody, anti-PD-1 antibody, anti-CD137 antibody, and anti-CTLA-4 antibody alone, respectively, or treated with the respective antibody-bound deformable particles according to the present disclosure having a particle size of 700 nm; FIG. 5B is a view showing a comparison of the cases in which the cells were treated with only two of the antibodies together, or treated with the deformable particles to which the two antibodies are bound; FIG. 5C is a view showing a comparison of cases in which the cells were treated with only three of the four antibodies together (left view: anti-PD-L1 antibody, CD137 antibody, and anti-CTLA-4 antibody; right view: anti-PD-1 antibody, anti-CD137 antibody, and anti-CTLA-4 antibody), or treated with the deformable particles to which the three antibodies are bound; FIG. 5D is a view showing a comparison of the cases in which the cells were treated with four antibodies alone, or treated with the deformable particles to which the four antibodies are bound. FIGS. 5E to 5F relate to the survival probability of liver cancer cells, FIGS. 5E and 5F are views showing a comparison of the cases in which the cells were treated with each of anti-PD-L1 antibody and anti-CTLA-4 antibody alone or treated with the antibody-bound deformable particles according to the present disclosure; FIG. 5G is a view showing a comparison of cases in which the cells were treated with only anti-PD-L1 antibody and anti-CTLA-4 antibody alone, or treated with the deformable particles, to which said two antibodies are bound, having a particle size of 700 nm.

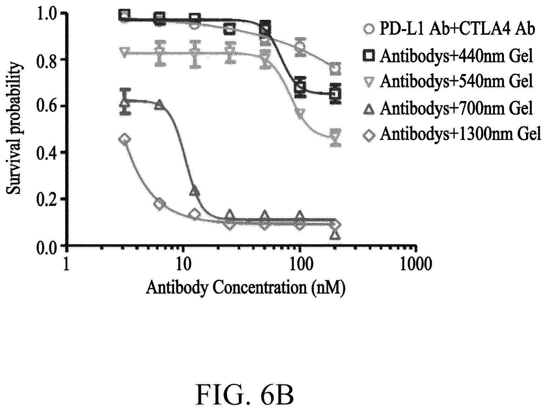

FIG. 6 is a view confirming the cancer cell killing effect according to the size of the deformable particle according to the present disclosure: FIG. 6A is a view confirming the cancer cell killing effect by binding an anti-CTLA-4 antibody to a hydrogel having a diameter of 440, 540, 700, or 1300 nm, respectively. FIG. 6B is a view confirming the cancer cell killing effect by binding an anti-PD-L1 antibody and anti-CTLA-4 antibody to a hydrogel having a diameter of 440, 540, 700, or 1300 nm, respectively.

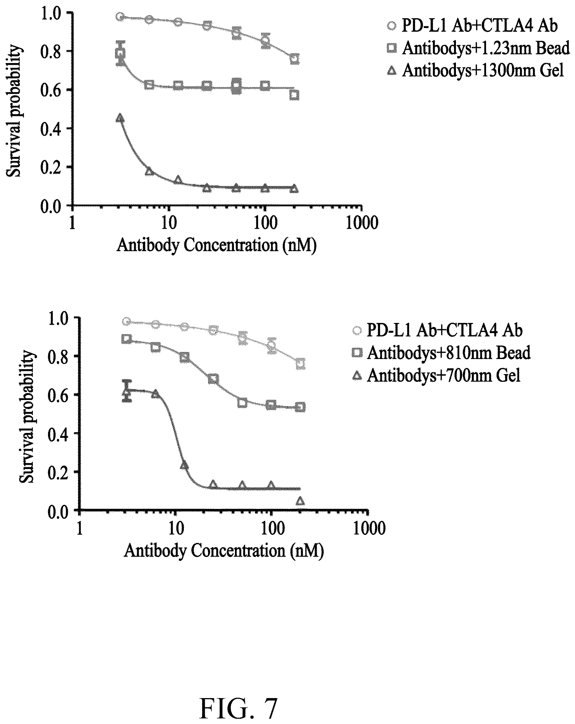

FIG. 7 is a result confirming the cancer cell killing effect according to antibody concentration in cases where cells were treated with the anti-PD-L1 antibody and the anti-CTLA-4 antibody together alone or treated with the anti-PD-L1 antibody and the anti-CTLA-4 antibody-bound deformable particles according to the present disclosure (hydrogel particles with the diameter of 700 or 1300 nm) and non-deformable polystyrene beads (particles with the diameter of 810 or 1230 nm), respectively.

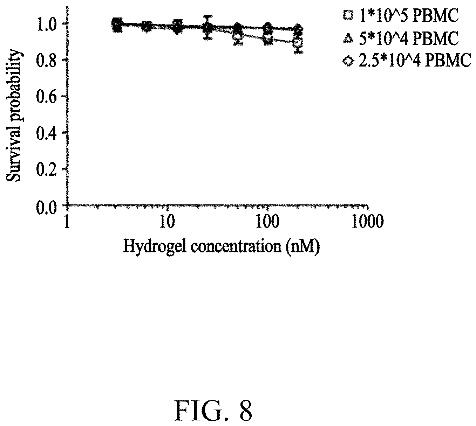

FIG. 8 is a view confirming the survival probability of cancer cells according to the concentration of hydrogel particles when cultured with immune cells without binding antibodies to deformable hydrogel particles according to the present disclosure.

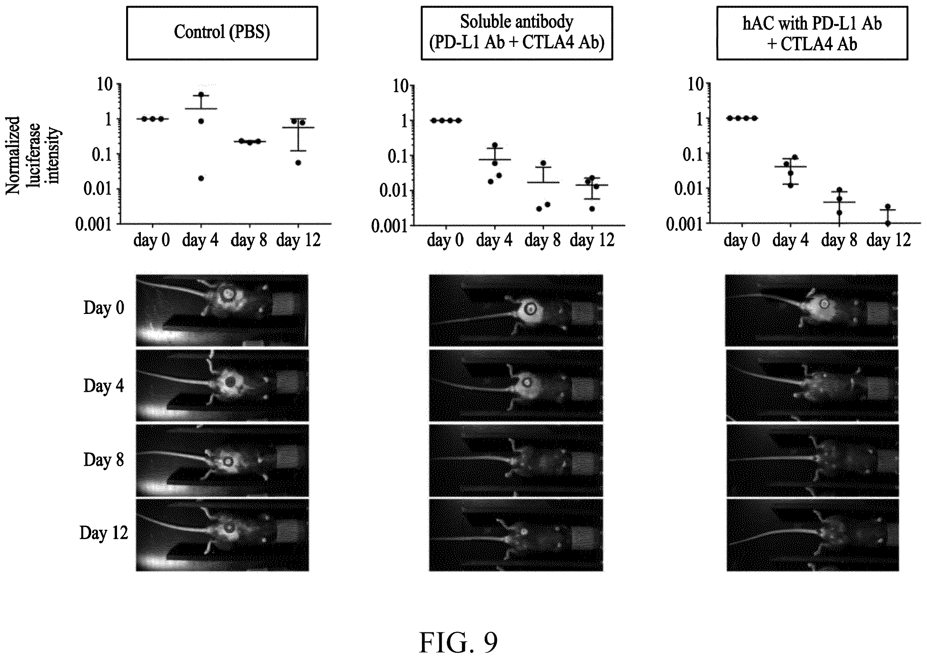

FIG. 9 is a result of evaluating the anticancer efficacy of deformable hydrogel particles according to the present disclosure in a mouse model, in which the fluorescence expression levels of cancer cells are shown on days 0, 4, 8, 12, and 16 after antibody injection in a group of mice treated only with PBS without injection of an antibody or hydrogel (control group); a group of mice treated with anti-PD-L1 antibody and anti-CTLA-4 antibody together alone; and a group of mice treated with an anti-PD-L1 antibody and an anti-CTLA-4 antibody-bound deformable particles (700 nm in diameter) according to the present disclosure.

### Best Mode for Carrying Out the Invention

Hereinafter, example embodiments are described in detail with reference to the accompanying drawings. However, since various changes may be made to the example embodiments, the scope of the patent application is not limited or limited by these example embodiments. It should be understood that all modifications, equivalents, or substitutes for the example embodiments are included in the scope of the rights.

The terms used in the example embodiments are used for the purpose of description only and should not be construed as limiting. The singular expression includes the plural expression unless the context clearly dictates otherwise. It should be understood that in the specification, terms such as "comprises" or "have" are intended to designate that a feature, number, step, operation, component, part, or a combination thereof described in the specification exists, and the possibility of the existence or addition of one or more other features or numbers, steps, operations, components, parts, or combinations thereof is not precluded in advance.

Unless defined otherwise, all terms used herein, including technical or scientific terms, have the same meaning as commonly understood by one of ordinary skill in the art to which the example embodiment belongs. Terms such as those defined in commonly used dictionaries should be interpreted as having a meaning consistent with the meaning in the context of the related art and should not be interpreted in an ideal or excessively formal meaning unless explicitly defined in the present application.

In addition, in the description with reference to the accompanying drawings, the same components are assigned the same reference numerals regardless of the reference numerals, and the overlapping description thereof is omitted. In describing the example embodiment, if it is determined that a detailed description of a related known technology may unnecessarily obscure the gist of the example embodiment, the detailed description thereof is excluded.

Components having functions in common with components included in one example embodiment are described using the same names in other example embodiments. Unless otherwise stated, the descriptions described in one example embodiment may be applied to other embodiments as well, and detailed descriptions within the overlapping range are excluded.

It is considered that all numerical ranges set forth throughout this specification include their upper limit and lower limit values, as well as all each numerical and narrower numerical range falling within that range, and each such numerical and narrower numerical range is expressly and specifically described herein.

According to one aspect of the present invention, there is provided a hydrogel particle characterized in that a protein capable of binding to a cell surface component of cancer cells and/or T-cells is bound to the surface and is deformable.

The term "cell surface component" according to the present disclosure refers to a protein located on a cell membrane and capable of binding or interacting with a component outside the cell. Exemplary cell surface components can comprise at least one selected from CD2, CD3, CD19, CD24, CD27, CD28, CD31, CD34, CD45, CD46, CD80, CD86, CD133, CD134, CD135, CD137, CD160, CD335, CD337, CD40L, ICOS, GITR, HVEM, Galectin 9, TIM-1, LFA-1, PD-L1, PD-L2, B7-H3, B7-H4, ILT3, ILT4, PD-1, CTLA-4, BTLA, MHC-I, MHC- II, TGF-β receptor, latent TGF-β-binding protein (LTBP), delta-like ligand (e.g., DLL-Fc, DLL-1 or DLL-4), WNT3, stem cell factor and thrombopoietin. More specifically, examples are immune checkpoint proteins such as PD-1 and CTLA-4 proteins on the surface of T-cells and PD-L1 and B7 on the surface of cancer cells.

The term "deformable" according to the present disclosure means that the physical shape of the particle can be changed and means a property in which it spontaneously changes the shape of the particle due to its softness when the hydrogel according to the present disclosure comes into contact with a cell. In particular, this means that when it comes into contact with other cells, the hydrogel, which had a spherical shape in body fluid, stretches thinly and widely without being broken or destroyed to have a formation that covers the cells like a blanket.

The degree of deformability of the hydrogel according to the present disclosure may be determined by measuring the change in diameter before and after the hydrogel particles contact the cell. Compared with the diameter (D) of the height axis of the hydrogel in the spherical state (horizontal X-axis, vertical Y-axis, and height Z-axis) before contacting the cell, it is determined to be deformable when the diameter of the height axis decreases by about 30 to about 99% (that is, the diameter of the height axis is 0.01 D to 0.7 D after contact) in a state in which the hydrogel contacts the cell and covers the cell like a blanket. Preferably, the diameter of the height axis after contacting the hydrogel is reduced by about 30 to about 90% compared to before contacting % (that is, the diameter of the height axis is about 0.1 D to about 0.7 D after contact).

The hydrogel according to the present disclosure has an appropriate softness and/or (visco) elastic modulus to be deformable at the cell surface. The hydrogel according to the present disclosure is soft, that is, has high ductility. In one aspect for these properties, the elastic modulus of the hydrogel particles at 25°C may be in the range of 0.01 Pa to 100 Pa (for a further discussion of the softness of hydrogels, see the following documents, which are incorporated herein by reference in their entirety: Mattias Karg et al., Langmuir 2019, 35, 6231-6255).

The diameter of the hydrogel according to the present disclosure may range from about 50 nm to about 3,000 nm, for example, about 100 nm to about 2,500 nm, or about 300 nm to about 2,000 nm, preferably 440 nm or more, more preferably about 540 nm or more, even more preferably 700 nm or more, and for example, about 700 nm to about 1,300 nm.

The diameter of the hydrogel can be measured by a conventional method known to those skilled in the art, for example, dynamic light scattering (light correlation spectroscopy, laser diffraction, low-angle laser light scattering (LALLS), and medium-angle laser light scattering (MALLS), light obscuration methods (such as the Coulter analysis method), or other methods (such as rheology, and light or electron microscopy).

The term "protein" according to the present disclosure refers to a high molecular weight substance formed by peptide bonds of several amino acids and includes an antibody, a recombinant protein, a peptide, a polypeptide, a glycoprotein, lipoprotein, synthetic protein, and the like, but are not limited thereto.

The term "antibody" according to the present disclosure refers to an immunoglobulin molecule having immunological reactivity with a specific antigen and refers to a protein molecule serving as a receptor that specifically recognizes an antigen. It encompasses both of a whole antibody and antibody fragments such as antigen-binding fragments.

In the present specification, "treatment alone" means that the antibody is applied by diluting the isolated antibody in PBS, etc. without binding it to the surface of a hydrogel or the like when cancer cells are treated with the antibody.

The term "recombinant protein" according to the present disclosure refers to a protein expressed from DNA engineered through recombinant DNA technology, and in particular, it may be expressed by cloning the recombinant DNA into an expression system such as a vector.

The term "hydrogel" according to the present disclosure means a three-dimensional network in which hydrophilic polymer chains are cross-linked as known per se in the art, for example, a hydrogel disclosed in Korean patent No. 10-1754774.

The surface of the hydrogel particle according to the present disclosure may be modified so that a protein may be bound to the surface thereof. In one example, the particle surface can be modified using standard bonding methods including maleimide/thiol and EDC/NHS bonding. More specifically, the linkage can comprise one formed by at least one reaction selected from the group consisting of carbodiimide cross-linking, Schiff base cross-linking, Azlactone cross-linking, carbonyl diimidazole (CDI) cross-linking, iodoacetyl cross-linking, hydrazide cross-linking, Mannich cross-linking, and maleimide cross-linking. Other useful methods of binding to hydrogel particles are described in the following document: Hermanson et al., (2013) Bioconjugate Techniques: Academic Press, which is incorporated herein by reference in its entirety.

According to another aspect of the present invention, provided are a pharmaceutical composition for treating cancer, the composition including the deformable particle, and a method of treating cancer by administering the pharmaceutical composition to a patient.

Here, "cancer" includes all cancers, and can comprise lung cancer, esophageal cancer, thymus cancer, breast cancer, liver cancer, stomach cancer, colorectal cancer, pancreatic cancer, cervical cancer, skin cancer, prostate cancer, ovarian cancer, thyroid cancer, bladder cancer, cervical cancer, bone marrow cancer, and biliary tract cancer but are not limited thereto.

The term "treatment" according to the present disclosure may be construed to include any action for improving or benefiting cancer symptoms by administering the pharmaceutical composition of the present invention to a patient but is not particularly limited thereto.

The pharmaceutical composition of the present invention may further comprise suitable carriers, excipients, and diluents commonly used in the preparation of pharmaceutical compositions, wherein said carriers may be non-natural carriers.

The carrier, excipient, and diluent can comprise lactose, dextrose, sucrose, sorbitol, mannitol, xylitol, erythritol, maltitol, starch, gum acacia, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate, and mineral oil.

Meanwhile, the pharmaceutical composition of the present invention is formulated and used in the form of oral dosage forms such as powders, granules, tablets, capsules, suspensions, emulsions, and syrups, aerosols, external preparations, suppositories, or sterile injection solutions according to conventional methods, respectively. In the case of formulation, it is formulated together with commonly used diluents, excipients, or carriers such as fillers, extenders, binders, wetting agents, disintegrants, and surfactants.

The pharmaceutical composition of the present invention may be administered as an individual therapeutic agent or may be administered in combination with other therapeutic agents. It may be administered sequentially or simultaneously with conventional therapeutic agents and may be administered in a single dose or multiple doses. In consideration of all of the above factors, it is important to administer an amount capable of obtaining the maximum effect with a minimum amount without side effects.

Meanwhile, the term "administration" in the present specification refers to introducing the pharmaceutical composition of the present invention to a subject by any suitable method, and the administration route may be administered through various routes as long as it can reach the target tissue.

For example, the administration route can comprise oral, parenteral, subcutaneous, intraperitoneal, intrapulmonary, or intranasal administration, and parenteral injection includes conventional parenteral administration methods such as intramuscular, intra-articular, intrathecal epidural, intravenous, intradermal, intraperitoneal, intratumoral, or subcutaneous administration. For parenteral administration of the composition, it is preferable to prepare a unit dosage formulation by mixing a pharmaceutically acceptable carrier, that is, one that is non-toxic to the receptor at the concentration and dosage used and that is miscible with other formulation ingredients under the desired purity.

The pharmaceutical composition of the present invention may be administered in "an immunologically effective amount," "an anti-tumor effective amount," "a tumor-inhibiting effective amount," or "a therapeutically effective amount." The exact amount of the composition of the present invention to be administered can be determined by a physician in consideration of individual differences in age, weight, tumor size, degree of infection or metastasis, and the condition of the patient (subject). In one example embodiment, the pharmaceutical composition of the present invention may be administered to the patient with a dosing cycle of 2 to 3 weeks in a dose of about 0.01 to about 20 mg/kg (patient body weight), about 0.01 to about 3 mg/kg, about 0.05 to about 2 mg/kg, about 0.1 to about 2 mg/kg, about 0.1 to about 1 mg/kg, about 1 to about 2 mg/kg, or about 2 to about 20 mg/kg. However, it is possible to administer a much smaller effective amount of the immuno-oncology agent compared to the case where the conventional immuno-oncology agent is administered alone without binding to the hydrogel according to the present invention. The optimal dosage and dosing regimen for a particular patient can be readily determined by one of ordinary skill in the pharmaceutical arts by monitoring the patient for signs of disease and adjusting treatment accordingly.

Hereinafter, the mechanism of the deformable particle according to the present disclosure is described in more detail with reference to the accompanying drawings.

FIG. 1 is a view for explaining the mechanism of the conventional immuno-oncology agent. There are many immune checkpoint proteins, PD-L1 and B7 proteins on the surface of cancer cells. When PD-L1 or B7 on the surface of cancer cells binds to PD-1 or CTLA-4 protein on the surface of T-cells, the immune function of T-cells is suppressed, and cancer cells evade the immune surveillance system. An immuno-oncology agent prevents T-cells from being inactivated and allows T-cells to attack cancer cells, thereby preventing the cancer cell's immune evasion mechanism. To this end, the immuno-oncology agent includes an anti-PD-L1 and/or an anti-CTLA-4 antibody. The anti-PD-L1 antibody binds to the PD-L1 protein on the surface of cancer cells to block the binding of PD-L1 and PD-1, and the anti-CTLA-4 antibody binds to the CTLA-4 protein of T-cells to block the binding of CTLA-4 and B7. Thus, these immune checkpoint proteins prevent the inactivation of T-cells, but instead activate the immune function so that the T-cells attack the cancer cells. However, in order to effectively prevent the immune function evasion of cancer cells, the conventional immuno-oncology agent should contain a higher or similar number of antibodies compared to the number of the PD-L1 protein on the cancer cell surface or the CTLA-4 protein on T-cells.

Meanwhile, the deformable particles according to the present disclosure can more effectively prevent immune evasion of cancer cells even with a small amount of antibody by using the softness of the hydrogel. The deformable particle according to the present disclosure contains a relatively small number of anti-PD-L1 antibodies (left side view of FIG. 2) compared to a conventional immuno-oncology agent (FIG. 1). The anti-PD-L1 antibody on the surface of the deformable particle binds to the PD-L1 protein on the surface of cancer cells. At the same time as binding, the hydrogel covers the surface of the cancer cell like a blanket, preventing the PD-1 protein of the T-cell from binding to the PD-L1 protein on the surface of the cancer cell (region inhibition) (right side view of FIG. 2). In other words, since the soft hydrogel deforms when attached to cancer cells and increases the contact area with cancer cells, the possibility of binding the checkpoint protein of cancer cells to the checkpoint protein of T-cells is greatly reduced even with a small amount of antibody, thereby effectively suppressing the immune evasion ability of cancer cells.

FIG. 3 is a comparison between a cancer cell therapeutic agent using the deformable particle according to the present disclosure and the conventional technology. A number of PD-L1 and B7 proteins exist on the surface of cancer cells, and when they bind to PD-1 or CTLA-4 proteins on the surface of T-cells, the immune function of T-cells is inhibited, and cancer cells evade the immune surveillance system (left side view of FIG. 3). Conventional immuno-oncology agents use antibodies to inhibit the immune evasion ability of cancer cells. For effective blocking, the conventional immuno-oncology agents should contain more or a similar number of anti-PD-L1 antibodies than PD-L1 protein on the surface of cancer cells. (Middle view of FIG. 3). On the other hand, the deformable particles according to the present disclosure contain antibodies to immune checkpoint proteins such as PD-1, PD-L1, CTLA-4, and/or CD137, and thus can be attached to cancer cells and/or T-cells via these antibodies. In addition, it can be attached to a larger area of the cell while changing its shape due to the properties of the soft hydrogel when attached to the cell (Right side view of FIG. 3). Soft hydrogels attached to large areas of cancer cells and/or T-cell surfaces prevent the inactivation of checkpoint proteins in T-cells by themselves.

Furthermore, the deformable particles according to the present disclosure may bind immune checkpoint proteins PD-1, PD-L1, and CTLA-4 targeting antibodies and T-cell activating receptor CD137(4-1BB) targeting antibodies alone to a hydrogel. In addition, the deformable particles combine and bind two or more of these antibodies together to the hydrogel, thereby achieving the effect of a multi-specific antibody such as a bispecific- or trispecific antibody (right side view of FIG. 4). That is, for the deformable particle according to the present disclosure, a hydrogel with multi-specificity can be easily produced using existing antibodies, thereby significantly reducing the development time and cost of multi-specific antibody synthesis.

Hereinafter, an experimental process and its results are described to compare the cancer cell survival probability when cancer cells are treated with an antibody alone or with an antibody-bound deformable particles according to the present disclosure and then are cultured with immune cells, peripheral blood mononuclear cells (PBMCs) together. The following examples are described for the purpose of illustrating the present invention, but the scope of the present invention is not limited thereto.

### Example

### Example 1: Preparation of hydrogel

Korean Patent No. 10-1754774 is referred for a method of preparing a hydrogel. The contents of this document are incorporated herein by reference in their entirety.

The hydrogel constituting the deformable particles according to the present disclosure can generally be prepared through the following steps: mixing 50 to 97.9% by weight of the main monomer, 2 to 40% by weight of the comonomer, and 0.1 to 10% by weight of a cross-linking agent so that the sum of the three components is 100% by weight; heating an aqueous solution including the monomer to 55 to 80°C; initiating the polymerization reaction with or without addition of an initiator; obtaining an aqueous hydrogel solution produced according to the reaction; and dialyzing the aqueous hydrogel solution with purified water for about 2 weeks.

The main monomer, comonomer, and cross-linking agent may be mixed in a content range of 50 to 97.9% by weight, 2 to 40% by weight, and 0.1 to 10% by weight, respectively. If the content of the main monomer is less than 50% by weight, polymerization reactivity may be lowered and polymerization may not occur well, whereas if it exceeds 97.9% by weight, it may be difficult to bind the required protein (for example, EDC/NHS coupling through acrylic acid) due to the lack of active groups provided by the comonomer. In each case where the content of the comonomer is less than 2% by weight or exceeds 40% by weight, it is technically difficult to bind a protein using the active group of the comonomer, or it is difficult to polymerize uniform hydrogel particles. In addition, when the content of the cross-linking agent is less than 0.1% by weight, it may be difficult to form a hydrogel, and when it exceeds 10% by weight, the softness of the hydrogel may be inhibited.

As one preparation example, the main monomer can comprise one selected from the group consisting of N-isopropylacrylamide, N-acryloylglycinamide, hydroxypropylcellulose, vinylcaprolactame, N-vinyl pyrrolidone, 2-hydroxyethyl methacrylate, ethylene glycol; amino acids such as aspartic acid, glutamic acid, and L-lysine; caprolactone, and vinyl methyl ether. The comonomer can comprise one selected from the group consisting of allylamine (AA), dimethylaminoethyl methacrylate (DMAEMA), dimethylaminoethyl acrylate (DMAEA), acrylic acid (AAc), ethylene glycol (EG), and methacrylic acid (MAAc).

In another preparation example, N-isopropylacrylamide may be used as the main monomer, and acrylic acid may be used as the comonomer. In this case, the cross-linking agent may be N, N'-methylene-bis-acrylamide (MBA).

In additional preparation example, the hydrogel can comprise at least one selected from the group consisting of poly(N-isoprophylacrylamide-co-allylamine) (poly(NIPAM-co-AA)), poly(N-isopropylacrylamide-co-2-(dimethylamino)ethyl methacrylate) (poly(NIPAM-co-DMAEMA)), poly(N-isopropylacrylamide-co-2-(dimethylamino)ethyl acrylate) (poly(NIPAM-co-DMAEA)), poly(N-isopropylacrylamide-co-acrylic acid) (poly(NIPAM-co-AAc)), poly(N-isopropylacrylamide-co-polyethylene glycol-acrylic acid) (poly(NIPAM-co-PEG-AAc)), and poly(N-isopropylacrylamide-co-methacrylic acid) (poly(NIPAM-co-MAAc)).

Ammonium persulfate (APS) may be used as the initiator, but it may be appropriately selected according to known techniques according to the type of monomer used.

In the hydrogel according to the present disclosure, the type and content of the monomer, cross-linking agent, and/or surfactant are controlled, or the polymerization initiation temperature and polymerization temperature are controlled according to common knowledge in the field of hydrogel production, thereby controlling the physical properties such as softness and size of the prepared hydrogel particles. For example, as the content of acrylic acid in the polymer is high and the content of BIS is low, the degree of deswelling of the prepared hydrogel particles tends to increase. In addition, if the content of BIS is too high, a hydrogel having a large particle size is not formed. In particular, the particle size of the hydrogel according to the present disclosure is preferably about 440 nm or more. In the preparation process, the size of the prepared particles can be adjusted in the above range by adjusting the amount and type of the surfactant and cross-linking agent, or by controlling the polymerization initiation temperature and polymerization temperature.

### Specific preparation example 1

### Preparation of poly(N-isopropylacrylamide-co-acrylic acid) hydrogel particles with a particle size of 700 nm

After dissolving 996 mg of N-isopropylacrylamide, 30.8 mg of BIS, and 65.5 mg of Tween80 in 100 ml of distilled water at room temperature, the solution was put in a 250 mL three-necked glass reactor. The oxygen in the solution was removed through the injection of argon gas for 1 hour, and at the same time, it was heated to a reaction temperature of 70°C using a heater. Then, 72 mg of acrylic acid was added to the solution in the reactor, argon gas was injected for 10 minutes, and the solution was heated to 70°C. Thereafter, 22.8 mg of ammonium persulfate was added to initiate polymerization. The polymerization reaction was completed by injecting argon gas and maintaining the reaction temperature at 70°C through heating while mixing the solution well using a magnetic bar for 6 hours. The resulting aqueous hydrogel solution was dialyzed by exchanging purified water twice a day for 2 weeks to remove unreacted monomers and surfactants. Then, hydrogel particles in powder form were prepared using a freeze dryer. For use, the freeze-dried hydrogel particles were dissolved in a solution (buffer or purified water) after measuring the mass in each experiment.

### Measurement of size of the prepared hydrogel particles

The size of the prepared hydrogel was measured by diluting the hydrogel in deionized water, and then calculating the average value of the values obtained by repeating measurement 5 times at 25°C using dynamic light scattering (DLS) equipment.

### Specific preparation example 2

### Preparation of poly(N-isopropylacrylamide-co-acrylic acid) hydrogel particles with a particle size of 440 nm

After dissolving 996 mg of N-isopropylacrylamide, 30.8 mg of BIS, and 28.8 mg of SDS in 100 ml of distilled water at room temperature, the solution was put in a 250 mL three-necked glass reactor. The oxygen in the solution was removed through the injection of argon gas for 1 hour, and at the same time, it was heated to a reaction temperature of 70°C using a heater. Then, 72 mg of acrylic acid was added to the solution in the reactor, argon gas was injected for 10 minutes, and the solution was heated to 70°C. Thereafter, 22.8 mg of ammonium persulfate was added to initiate polymerization. The polymerization reaction was completed by injecting argon gas and maintaining the reaction temperature at 70°C through heating while mixing the solution well using a magnetic bar for 6 hours. The resulting aqueous hydrogel solution was dialyzed by exchanging purified water twice a day for 2 weeks to remove unreacted monomers and surfactants. Then, hydrogel particles in powder form were prepared using a freeze dryer. For use, the freeze-dried hydrogel particles were dissolved in a solution (buffer or purified water) after measuring the mass in each experiment.

### Measurement of size of prepared hydrogel particles

The size of the prepared hydrogel was measured by diluting the hydrogel in deionized water, and then calculating the average value of the values obtained by repeating measurement 5 times at 25°C using dynamic light scattering (DLS) equipment in the same manner as in specific preparation example 1.

### Example 2: Modification of surface of hydrogel particles: EDC/NHS coupling

EDC/NHS coupling was performed so that the antibody could be bound to the hydrogel particles obtained in Example 1 above.

EDC (1-ethyl-3-(3-dimethylaminopropyl) carbodiimide hydrochloride) (SIGMA-ALDRICH; Cat. E7750-25G) and NHS (N-Hydroxysuccinimide) (SIGMA-ALDRICH; Cat. 130672-25G) were used as cross-linking agents. 0.1 M MES buffer (2-[N-morpholino]ethanesulfonic acid buffer) (Biosolution; Cat. BM020-5.5) was used as a cross-linking agent solvent.

5 mg of poly(N-isopropylacrylamide-co-acrylic acid) hydrogel particles (acrylic acid content of 10%) were mixed with 500 µl of 0.1 M MES buffer to prepare a hydrogel mixture, and 4 mg of EDC and 8 mg of NHS, respectively, were dissolved in 200 µl of 0.1 M MES buffer. Then, 400 µl of hydrogel mixture, 200 µl of EDC lysate, and 200 µl of NHS lysate were all combined, and the mixture was cultured for 5 minutes at room temperature. After culture, 16 µl of 500 µM Protein A (Sino Biological Inc.; LC12NO0802) was added and mixed. The mixture was cultured for an additional 1 hour at room temperature while inverting the tube containing the sample.

After culture, the sample was centrifuged at 8000 rpm for 2 minutes at room temperature, the supernatant was removed in a clean bench, and 500 µM of PBS (WEL GENE; LB001-02) was added to wash the sample. The centrifugation and PBS washing were repeated 5 or more times to remove all of the MES buffer and unreacted substances (EDC, NHS, protein A) in the sample. After the last centrifugation, the supernatant was removed. The sample was suspended in 800 µM PBS, and then the sample was stored at 4°C.

### Example 3: Binding of antibodies to hydrogel particles

The process of binding the antibody to the surface-modified hydrogel particles obtained in Example 2 to complete the deformable particles of the present disclosure is described. Anti-PD-L1 antibody (Sino Biological; Cat. 10084-R639); anti-PD-1 antibody (Sino Biological; Cat. 10377-HN94); anti-CD137 antibody (Sino Biological; Cat. 10041-RP01); and anti-CTLA-4 antibody (Bioxcell; Cat. BE0190, Lot. 744719s1) were used, all of which were human antibodies.

The antibody was added to the protein A-modified hydrogel particles to have a concentration of 8 µM, and the mixture was cultured at 4°C for 1 hour to induce binding of the antibody to the hydrogel particles. DMEM (WEL GENE; Cat. LM001-11; containing 10% FBS and 1% antibiotic-antimycotic (A-A)) was added so that the top concentration of the antibody was 400 nM when the cells were treated with the antibody-binding hydrogel (i.e., deformable particles according to the present disclosure). In order to prepare several samples with different antibody concentrations, based on the volume of the antibody and hydrogel used in preparing the solution with the top concentration, 1XPBS was added instead of the antibody. It was serially diluted so that the antibody concentrations were 400 nM, 200 nM, 100 nM, 50 nM, 25 nM, 12.5 nM, and 6.25 nM, respectively. The completed deformable particles were treated at a rate of 100 µl per cell in the subsequent process. Since the 48-well plate to which the deformable particles were added had 100 µl of medium per well, the concentration of the added antibody was eventually diluted in half. Thus, the final concentrations of antibody added to the cells were 200 nM, 100 nM, 50 nM, 25 nM, 12.5 nM, 6.25 nM, and 3.125 nM, respectively. When two or more antibodies were combined to bind to the hydrogel particles, the total concentration of the antibody was prepared as above, but the ratio of each antibody was equal (i.e., a ratio of 1 : 1).

Three groups were additionally prepared for comparative experiments: (i) the PBS group was prepared at a concentration of 10% v/v (DMEM 360 µl + PBS 40 µM) as a control that did not contain both the antibody and the hydrogel, (ii) the antibody-non-binding hydrogel group was prepared by mixing PBS without antibody in a hydrogel having the same volume as the hydrogel volume used to match the top concentration of antibody (200 nM) in the process of preparing the deformable particles described above (that is, the process of binding antibody to hydrogel), and (iii) the antibody group not bound to the hydrogel was prepared by diluting the antibody in DMEM to have a concentration of 400 nM, 200 nM, 100 nM, 50 nM, 25 nM, 12.5 nM, or 6.25 nM, then cells were treated with 100 µl, and as described above, the antibody concentration was consequently diluted in half by the medium contained in each well. Therefore, the final concentrations of the antibody added to the cells were 200 nM, 100 nM, 50 nM, 25 nM, 12.5 nM, 6.25 nM, and 3.125 nM, respectively.

### Example 4: In vitro analysis of survival probability of cancer cell

In order to verify whether the deformable hydrogel particles according to the present disclosure inhibit immune evasion of cancer cells and promote cancer cell death by immune cells, an *in vitro* experiment was conducted to check the survival probability of cancer cells.

### Preparation of cancer cells for analysis experiment of survival probability of cancer cells

On the 0th day of the experiment, a breast cancer cell line, MCF-7 (Korea Cell Line Bank, Seoul National University) was prepared. The existing medium was removed from the T75 flask culturing MCF-7, and the remaining medium was washed with 10 ml of PBS and then removed by suctioning the PBS. After treatment with 2 ml of 0.25% trypsin-EDTA (Gibco; REF. 25200-056), it was cultured at 37°C for 2 minutes. Trypsin-EDTA was neutralized by adding 8 ml of DMEM containing 10% Fetal bovine serum (FBS) (Gibco; Lot. 1985900) and 1% antibiotic-antimycotic (A A) (Gibco; REF. 15240-062). Then, cancer cells separated from the bottom of the flask were collected and transferred to a 15 ml tube. Then, centrifugation was performed for 3 minutes at 24°C and 1,300 rpm. After removing the supernatant from the centrifuged sample, 5 ml of DMEM was added to suspend the cell pellet. They were inoculated into a 48-well plate at a concentration of 1 × 10⁴ cells/100 µl/well. Cells were cultured overnight at 37°C.

### Treatment of antibody-bound deformable particles and PBMCs

After removing 100 µl of the medium from the cultured cancer cells, each well was treated with 100 µl of the deformable particles to which the antibody prepared in Example 3 having various concentrations was bounded. The cells were cultured at 37°C for 2 hours. As a control, (i) PBS group, (ii) antibody-not-bound hydrogel group, and (iii) antibody group not bound to the hydrogel prepared in Example 3 were also treated at 100 µl per well and cultured under the same conditions.

While treating cancer cells with the deformable hydrogel particles, peripheral blood mononuclear cells (PBMCs) (Zenbio; Cat. SER-PBMC-200P-F) composed of T-cells, B-cells, NK cells, etc. were prepared. PBMCs stored at -70°C were thawed in a 37°C water bath. The thawed cells were transferred to a 50 ml tube and then RPMI medium (Gibco; Lot. 2145483) (10% FBS and 1% A-A was added) was added so that the total volume was 25 ml. After centrifugation at 400 g for 10 minutes at 19°C, the supernatant was removed, and 5 ml of RPMI was added to suspend the cell pellet.

The deformable hydrogel particle-treated cancer cells were treated with the prepared PBMCs at a concentration of 1 × 10⁴ cells/100 µl/well. Each well was treated with 10 µl of FBS, and the cells were cultured at 37°C and 5% CO₂ in an incubator for 4 days. During culture, 20 µl of DMEM and 10 µl of FBS were added to each well every other day.

### Confirmation of cancer cell survival probability through fluorescence staining

After culturing for 4 days, fluorescence staining was performed to check the cell survival probability on the 5th day of the experiment. A fluorescence staining reagent was prepared by mixing 5 µl of Calcein AM (Invitrogen; Lot. 2049068) per 10 ml of PBS.

The medium was removed from the cancer cells cultured with the deformable particles and PBMCs. After washing with PBS, PBS was also removed by suction. 200 µl of the prepared fluorescence staining reagent was treated in each well. Then, the 48-well plate was wrapped with foil to block light, and the cells were cultured at 37°C for 15 minutes. After culture, the fluorescence staining reagent was removed. After washing with PBS, 200 µl of DMEM medium was added per well, and fluorescence imaging was performed with a fluorescence microscope (Nikon; Ti2-E).

The captured fluorescence photos were analyzed using the Image J program (provided by the National Institutes of Health, USA). The area stained with Calcein AM compared to the total area was calculated and expressed as cancer cell survival probability.

### Experiment result

Hereinafter, the cancer cell killing effect of the deformable particles according to the present disclosure is compared with that of a conventional antibody-based immuno-oncology agent (that is, by treating the antibody alone), and it is confirmed whether the hydrogel size and softness of the deformable particles affect the cancer cell killing effect.

### Comparison of cancer cell killing effect of conventional immuno- cancer agent and deformable particle according to present disclosure

MCF7, a breast cancer cell, was treated with an antibody alone (that is, the antibody is present in a free state without being bound to a deformable particle) (hereinafter, "antibody group") or treated with an antibody-bound deformable particle according to the present disclosure (hereinafter, antibody + GEL group). Then, the cells were cultured with PBMC, an immune cell, and the survival probability was compared (FIGS. 5A to 5D). As the antibodies, anti-PD-L1 antibody, anti-PD-1 antibody, and anti-CTLA-4 antibody targeting immune checkpoint protein and anti-CTLA-4 antibody targeting T-cell activation receptor were used.

FIG. 5A shows the cancer cell survival probability according to the antibody concentration when the cells were treated with each of the above four antibodies alone or treated with each of said antibodies-bound deformable particles according to the present disclosure.

In the case of anti-PD-1 antibody, the antibody group and the antibody + GEL group showed cancer cell survival probabilities of 0.93 and 0.95, respectively at an antibody concentration of 3.125 nM. However, it was confirmed that the difference in cancer cell survival probability between the two groups was widened when the antibody concentration was 12.5 nM or higher. At the top concentration of 200 nM, the cancer cell survival probability of the antibody group was 0.5, whereas that of the antibody + GEL group had a significantly lower probability of 0.05.

In the case of anti-PD-L1 antibody, both the antibody group and the antibody + GEL group showed a similar cancer cell survival probability of 0.7 at an antibody concentration of 3.125 nM. However, it was confirmed that the difference in cancer cell survival probability between the two groups was widened when the antibody concentration was 6.25 nM or higher. At the top concentration of 200 nM, the cancer cell survival probability of the antibody group was 0.5, whereas that of the antibody + GEL group was 0.02.

In the case of anti-CD137 antibody, there was no significant difference in the cancer cell survival probability of the two groups at a concentration of 12.5 nM or less. However, it was confirmed that the cancer cell survival probability differed from when the antibody concentration was 12.5 nM or higher, and at 100 nM, the cancer cell survival probability showed the greatest difference by 8 times. At the top concentration of 200 nM, the cancer cell survival probability of the antibody group was 0.37, whereas that of the antibody + GEL group had a significantly lower probability of 0.04.

In the case of anti- CTLA-4 antibody, there was no significant difference in the cancer cell survival probability of the two groups at a concentration of 12.5 nM or less. However, it was confirmed that the cancer cell survival probability differed from when the antibody concentration was 12.5 nM or higher, and at 100 nM, the cancer cell survival probability showed the greatest difference by 8 times. At the top concentration of 200 nM, the cancer cell survival probability of the antibody group was 0.9, whereas that of the antibody + GEL group had a significantly lower probability of 0.15.

FIG. 5B shows the cancer cell survival probability according to the antibody concentration when the cells were treated with two of the above four antibodies alone or treated with the two antibodies-bound deformable particles.

In the combination of the anti-PD-L1 antibody and anti-PD-1 antibody, the antibody group maintained the cancer cell survival probability at 0.9 until the concentration of 25 nM, but showed a sharp decrease from 50 nM. The cancer cell survival probability of the antibody + GEL group was 0.55 at 3.125 nM, which was lower than that of the antibody group, and the survival probability was significantly reduced in a concentration-dependent manner. The concentration with the greatest difference in survival probability between the antibody group and the antibody + GEL group was 25 nM and 50 nM. At 200 nM, the antibody group showed a survival probability of 0.3, whereas the antibody + GEL group showed a survival probability of 0.04.

In the combination of the anti-PD-L1 antibody and anti-CTLA-4 antibody, it was confirmed that the antibody group showed a survival probability of 0.98 at a concentration of 3.125 nM and maintained 0.7 even at 200 nM. On the other hand, the cancer cell survival probability of the antibody + GEL group was 0.6 at 3.125 nM and decreased to 0.05 at 200 nM. The cancer cell survival probability at 3.125 nM of the antibody + GEL group was lower than that of the antibody group at 200 nM.

In the combination of the anti-PD-L1 antibody and anti-CD137 antibody, it was confirmed that the antibody group showed a survival probability of 0.97 at a concentration of 3.125 nM and maintained 0.6 at 200 nM. On the other hand, the antibody + GEL group showed a significantly lower survival probability of 0.9 at 3.125 nM and 0.02 at 200 nM.

In the combination of the anti-PD-1 antibody and anti-CD137 antibody, it was confirmed that the antibody group showed a survival probability of 0.9 at a concentration of 3.125 nM and maintained 0.7 at 200 nM. On the other hand, the cancer cell survival probability at 3.125 nM of the antibody + GEL group was 0.7, similar to the cancer cell survival probability at 200 nM of the antibody group, and at 200 nM, the survival probability was significantly lower to 0.06.

In the combination of the anti-PD-1 antibody and anti-CTLA-4 antibody, the antibody group showed a survival probability of 0.9 at a concentration of 3.125 nM, and maintained a survival probability of 0.7 even at 200 nM. The cancer cell survival probability at 3.125 nM in the antibody + GEL group was 0.6, which was lower than the cancer cell survival probability at 200 nM of the antibody group and was significantly lower to 0.05 at 200 nM. The concentration at which the survival probability of the two groups differed the most was 25 nM, and a 9-fold difference was observed.

FIG. 5C shows the cancer cell survival probability according to the antibody concentration when the cells were treated with three of the above four antibodies alone or treated with the three antibodies-bound deformable particles.

In the combination of the anti-PD-L1 antibody, anti-CD137 antibody, and anti-CTLA-4 antibody (left graph of FIG. 5C), the antibody group showed a survival probability of 0.9 at a concentration of 3.125 nM, and maintained a survival probability of 0.6 even at 200 nM. On the other hand, the antibody + GEL group already showed a survival probability of 0.7 at 3.125 nM, and the survival probability was significantly reduced to 0.1 at 200 nM.

In the combination of the anti-PD-1 antibody, anti-CD137 antibody, and anti-CTLA-4 antibody (right graph of FIG. 5C), the antibody group showed a survival probability of 0.9 at 3.125 nM, and maintained almost the same survival probability up to 100 nM, but then rapidly dropped to 0.4 at 200 nM. On the other hand, the antibody + GEL group already showed a survival probability of 0.24 at 3.125 nM, and the survival probability was significantly reduced to 0.02 at 200 nM.

FIG. 5D shows the cancer cell survival probability according to the antibody concentration when the cells were treated with all of the above four antibodies alone or treated with the four antibodies-bound deformable particles. The antibody group showed a survival probability of 0.99 at 3.125 nM and a cancer cell survival probability of 0.5 at 200 nM. On the other hand, the antibody + GEL group already showed a probability of 0.6 at 3.125 nM, particularly when the antibody concentration was 12.5 nM or higher, the survival probability was sharply decreased, and the survival probability was 0.06 at 200 nM.

In conclusion, it was confirmed that in all cases of treatment with each of the four antibodies, or a combination of two or more, cancer cell survival probability was significantly reduced when combined with deformable particles rather than treatment with the antibody alone. In particular, it was confirmed that the cancer cell killing effect of the deformable particles was more excellent when two or more antibodies were bound together than when a single antibody was bound. This proves that the deformable particles according to the present disclosure can promote cancer cell death by using a small amount of antibody compared to conventional immuno-oncology agents, as well as achieve the effect of multiple antibodies through a relatively simple preparation process.

In particular, it was confirmed that the cancer cell survival probability was almost unchanged in the group not treated with both the antibody and the deformable particles (PBS group), or the group treated with the deformable particles to which the antibody was not bound (antibody-not-bound hydrogel group). This means that the hydrogel itself does not significantly affect cancer cells or immune cells, but when combined with an antibody, it prevents immune evasion of cancer cells by blocking the interaction between the cancer cells and immune cells through the binding of the antibody to the surface proteins of the cells. As such, it is suggested that the hydrogel particles of the present invention do not simply act as a drug delivery means, but function like immune cells as artificial T-cells.

The IC₅₀ for cancer cell survival probability of the group treated with the antibody alone (antibody group) and the group treated with antibodies-bound deformable particles (antibody + GEL group) are shown in Table 1 below.

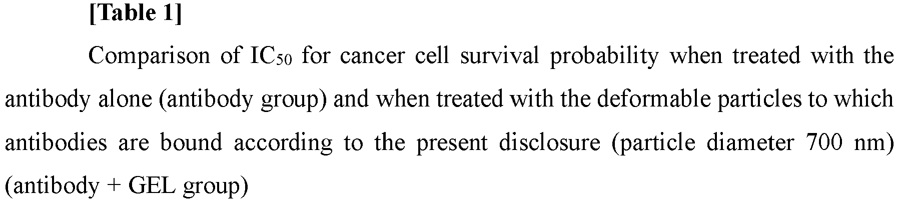

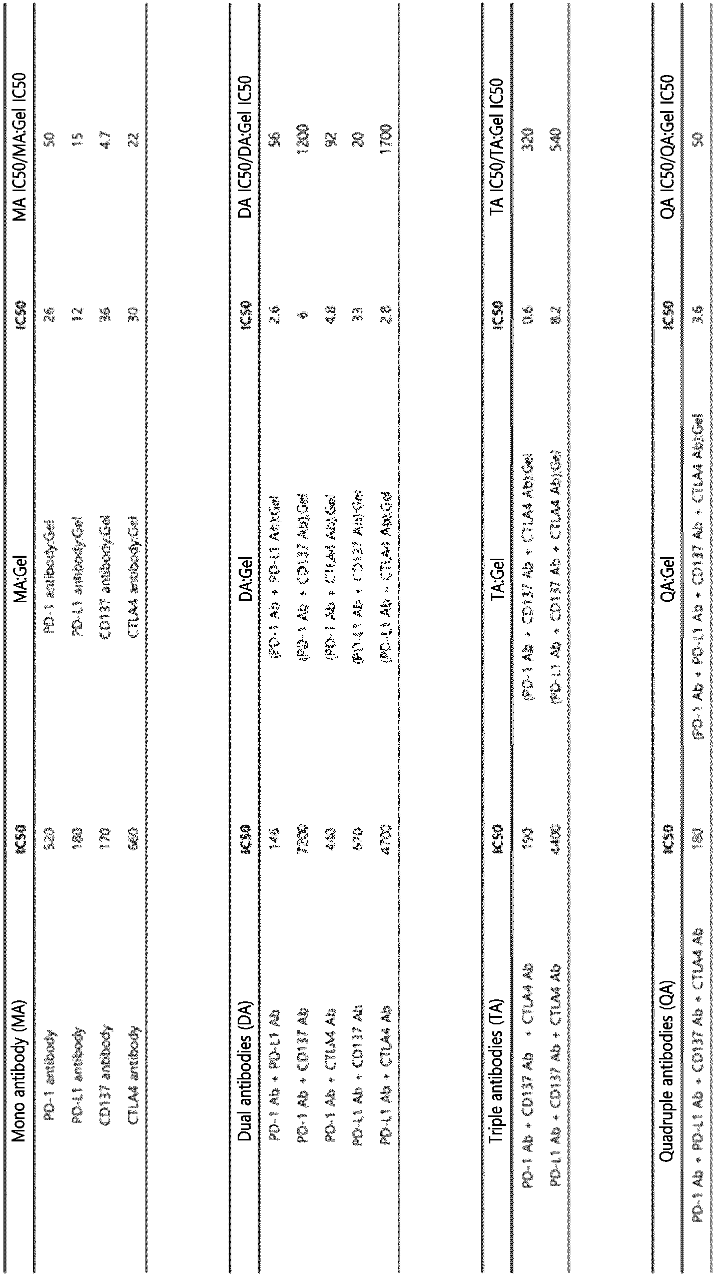

### Confirmation of killing effect of hydrogel of present disclosure on liver cancer cells

The same experiment as described above was also performed on liver cancer cells (HepG2), and it was confirmed that the hydrogel particles according to the present disclosure had a cancer cell killing effect similar to that in breast cancer cells. The results are shown in FIGS. 5E to 5G.

### Example 5: Comparison of cancer cell killing effect according to hydrogel size

A test was performed to confirm whether the hydrogel size of the deformable particles according to the present disclosure affected cancer cell death (FIG. 6). The diameter of the hydrogel to be described below was measured in deionized water and is the diameter of the hydrogel in a spherical state that is not bound to cells.

First, it was confirmed whether the size of the hydrogel affected the cancer cell survival probability in the deformable particles bound with a single antibody (FIG. 6). Each group of cancer cells was treated with anti-CTLA-4 antibody-bound hydrogels having a diameter of 440 nm, 540 nm, 700 nm, or 1,300 nm. A group of cancer cells was treated with anti-CTLA-4 antibody alone. Their cancer cell survival probabilities were compared.

As shown in FIG. 6A, it was confirmed that the cancer cell survival probability of the hydrogel having a diameter of 440 nm was slightly lowered compared to that of treatment with the anti-CTLA-4 antibody alone, but the cancer cell survival probability was not significantly lowered. However, when the diameter was 540 nm or more, the cancer cell survival probability of the antibody + GEL group was significantly lower than that of the antibody group by 20% or more at the same antibody concentration.

It was confirmed whether the size of the hydrogel particles affected the cancer cell survival probability even when two types of antibodies are bound to the hydrogel particles (FIG. 6B). Cancer cells were treated with anti-PD-L1 antibody and anti-CTLA-4 antibody-bound hydrogel particles having a diameter of 440 nm, 540 nm, 700 nm, or 1,300 nm. Cancer cells were also treated with anti-PD-L1 antibody and anti-CTLA-4 antibody alone. Their cancer cell survival probabilities were compared. As shown in FIG. 6B, it was confirmed that when the two antibodies were bound in combination, the hydrogel particles with a diameter of 440 nm also showed increased cancer cell killing effect compared to the antibody alone group, and when the hydrogel particles with a diameter of 700 nm or more were used, the cancer cell survival probability was significantly reduced.

These results show that the diameter of the hydrogel particles affects the cancer cell killing effect of the deformable particles according to the present disclosure. That is, the diameter of the hydrogel particles for the deformable particles according to the present disclosure to have superior cancer cell killing ability compared to conventional immuno-oncology agents is 440 nm or more, preferably 540 nm or more, and more preferably 700 nm or more.

### Example 6: Comparison of cancer cell killing effect according to presence or absence of softness of hydrogel

It was confirmed whether the softness of the hydrogel affected the cancer cell killing effect of the deformable particles according to the present disclosure (FIG. 7).

Anti-PD-L1 antibody and anti-CTLA-4 antibody were bound to polystyrene beads with a particle size of 1,230 nm [product name: CP-10-10 (Spherotech, Lake Forest, IL, USA)], and the same antibodies were bound to deformable particles (1,300 nm) of the present disclosure. The cancer cell killing abilities of them were compared. Polystyrene beads are made of an aromatic hydrocarbon polymer, and, unlike the deformable particles according to the present disclosure, are spherical non-deformable hard beads that have no softness.

As shown in FIG. 7, when treated with the antibody-bound deformable particles according to the present disclosure, the cancer cell survival probability was sharply decreased compared to the case of treating with the antibody alone. When treated with the antibody-bound polystyrene beads, there was no significant difference at a low concentration from the antibody group, and the cancer cell survival probability was as high as 0.6 even at the top concentration (200 nM).

The same experiments were repeated using the same antibodies but changing the particle size of polystyrene beads [product name: CP-08-10 (Spherotech, Lake Forest, IL, USA)] and hydrogel particles to 810 nm and 700 nm, respectively, and the results were similar to the previous experimental results (Bottom of FIG. 7).

These results demonstrate that the deformable particles according to the present disclosure cover a wider area of cells due to the softness of the hydrogel, thereby effectively blocking the interaction between cancer cells and T-cells and promoting cancer cell death.

### Comparative Example 1: Confirmation of cancer cell killing effect of deformable hydrogel particles alone

The cancer cell survival probability according to the concentration of hydrogel particles was confirmed when cultured with immune cells without binding antibodies to deformable hydrogel particles according to the present disclosure (FIG. 8). As confirmed in FIG. 8, when the antibody was not bound to the deformable hydrogel particles according to the present disclosure, the cancer cell killing ability was insignificant. This means that, in order for the hydrogel particles according to the present disclosure to have a cancer cell killing effect, it is essential that a protein capable of binding to a cell surface component (ideally, an immune checkpoint regulatory protein, an immune cell activation protein, or a cancer cell regulation protein) of cancer cells or T-cells is present on the hydrogel surface. Further, this suggests that the hydrogel particles according to the present disclosure act as artificial T-cells that mimic the function of T-cells to maintain the activity of immune cells in the human body and effectively inhibit the immune evasion mechanism of cancer cells.

### Example 7: Evaluation of anticancer efficacy of deformable hydrogel particles in mouse model

The anticancer efficacy of deformable hydrogel particles according to the present disclosure was evaluated in a mouse model. This experiment was conducted with approval from Korea University IACUC (KOREA-2020-0203). Mice were purchased from Orient Bio (located in Jungwon-gu, Seongnam-si, Gyeonggi-do, Korea), and 6-week-old females of C57BL/6 species were used. After being brought into the animal room, the experiment was carried out after a stabilization period for one week. For engraftment of cancer cells, cyclosporine (Chong Kun Dang, Cat. EG001, 450 ug/15 g) was administered by intramuscular injection (IM) from 2 days before cell inoculation, and ketoconazole (Eaglevet, Cat. 170432001, 2.5 ug/ul) was mixed in drinking water and then the mixture was administered. 2.5 × 10^6 cells of MCF-7-luc2 (ATCC, HTB-22-LUC2), a human breast cancer cell line, were mixed with 100 ul of serum free RPMI 1640 (Gibco, Cat. 11875093) + 100 ul of Matrigel (Corning, Lot. 0062015), and the mixture was inoculated by subcutaneous injection (SC) in the fourth nipple. Cyclosporine was additionally administered intramuscularly for 2 days after inoculation. To confirm the expression level of MCF-7/Luc2, fluorescence imaging was performed with an Indigo analyzer (NightOWL II LB 983). 15 minutes before shooting, 1.5 mg/100 ul of D-luciferin (Goldbio, GOLD-1G) was administered by SC, and luminescence was taken every 4 days.

The duration of the experiment was 16 days, and fluorescence imaging was performed 5 times in total. The day the cancer cells were inoculated was counted as day 0, and fluorescence imaging was performed on day 0, day 4, day 8, day 12, and day 16, respectively. The experimental groups were classified into a total of three groups: i) control group (injected with only cancer cells without hydrogel, PBS group), ii) dual antibody group (treated cancer cells with a PD-L1 antibody and a CTLA4 antibody, which are not bound to hydrogel, and iii) dual antibodies-bound hydrogel-treated group (treated with a hydrogel to which a PD-L1 antibody and a CTLA4 antibody are bound - hAC with PD-L1 ab + CTLA-4 ab prepared in Example 3). 3 or 4 mice, respectively, were used for each experimental group. After the injection of cancer cells, the control group was injected with PBS every 4 days, and the treatment group was treated with dual antibodies or hydrogels to which dual antibodies are bound every 4 days, respectively. On the last day, day 16, fluorescence imaging was performed without injection of PBS or drugs, and they were sacrificed using CO₂ to collect tissues.

For the fluorescence photograph taken, the fluorescence data value was extracted as the bioluminescence expression level (ph/s) in the program in the Indigo analysis equipment. The bioluminescence expression level was corrected for each individual and group based on the bioluminescence expression level on the 4th day after the start of drug treatment.

The sample concentration of the sample-treated group is as follows.
- Concentration of the dual antibody group: 100 ul was injected by calculating as 60 ug (30 ug of PD-L1 antibody + 30 ug of CTLA4 antibody)/20 g per mouse.
- Concentration of the dual antibodies-bound hydrogel group: 100 ul was injected by calculating as 60 ug (60 ug of PD-L1 antibody + 60 ug of CTLA4 antibody + hydrogel (included in the same amount as the amount of antibody))/20g per mouse. In this case, since the antibody was diluted with the hydrogel in a ratio of 1 : 1 due to the presence of the hydrogel, the concentration of each antibody was increased two-fold compared to the case in which the hydrogel was not present.

### Experiment result

FIG. 9 shows the amount of fluorescence of cancer cells analyzed in the fluorescence photograph. In the case of the control group in which only cancer cells were injected, and no antibody or hydrogel sample was injected (left view in FIG. 9), the fluorescence amount of cancer cells was averagely increased on day 4 after inoculation of cancer cells. The fluorescence amount of cancer cells was decreased on day 8 after inoculation of cancer cells, but increased again on day 12. It was confirmed that the fluorescence amount of cancer cells fluctuated without a constant direction. In the case of the dual antibody group (middle view in FIG. 9), the fluorescence amount of cancer cells was greatly reduced to 0.1 on day 4 after inoculation of the cancer cells, and the fluorescence amount of the cancer cells was decreased to 0.02 on day 8 after inoculation, but the fluorescence amount of cancer cells on day 12 was similar to that of day 8 so that the fluorescence amount of the cancer cells was not decreased anymore. In the case of the group treated with the two antibodies-bound hydrogel of the present disclosure (rightmost view in FIG. 9), the fluorescence amount of cancer cells was dramatically decreased to 0.04 on day 4 after inoculation of the cancer cells, the fluorescence amount of cancer cells was decreased to 0.004 on day 8 of inoculation, and the fluorescence amount of cancer cells was decreased to 0.001 and no cancer cells were observed in some subjects on day 12 of inoculation. Accordingly, it was confirmed that cancer cells were significantly reduced.

As described above, although the example embodiments have been described with reference to the limited drawings, those skilled in the art may apply various technical modifications and variations based on the above. Therefore, other implementations, other example embodiments, and equivalents to the claims are also within the scope of the following claims.

## Claims

1. A hydrogel particle being deformable and having a protein, which is capable of binding to a cell surface component of cancer cells or T-cells, bound to the surface of the hydrogel particle.

2. The hydrogel particle of claim 1, wherein the cell surface component is at least one selected from the group consisting of CD2, CD3, CD19, CD24, CD27, CD28, CD31, CD34, CD45, CD46, CD80, CD86, CD133, CD134, CD135, CD137, CD160, CD335, CD337, CD40L, ICOS, GITR, HVEM, Galectin 9, TIM-1, LFA-1, PD-L1, PD-L2, B7-H3, B7-H4, ILT3, ILT4, PD-1, CTLA-4, BTLA, MHC-I, MHC-II, TGF-β receptor, latent TGF-β-binding protein (LTBP); delta-like ligand including DLL-Fc, DLL-1, or DLL-4; WNT3, stem cell factor, and thrombopoietin.

3. The hydrogel particle of claim 1, wherein the cell surface component of the cancer cell is PD-L1 protein, and the cell surface component of the T-cell is selected from the group consisting of PD-1 protein, CTLA-4 protein, and CD137 protein.

4. The hydrogel particle of claim 1, wherein the protein bound to the surface of the hydrogel is an antibody, a recombinant protein, or a combination thereof.

5. The hydrogel particle of claim 4, wherein the antibody is an anti-PD-1 antibody, an anti-PD-L1 antibody, an anti-CD137 antibody, an anti-CTLA-4 antibody, or a combination thereof.

6. The hydrogel particle of claim 4, wherein the recombinant protein is at least one selected from the group consisting of a protein, an aptamer, or a combination thereof, and wherein the protein or aptamer is capable of targeting and binding at least one selected from the group consisting of PD-L1 protein, PD-1 protein, CTLA-4 protein, and CD137 protein.

7. The hydrogel particle of claim 1, wherein the diameter of the hydrogel in deionized water is 440 nm or more.

8. The hydrogel particle of claim 7, wherein the diameter of the hydrogel in deionized water is 540 nm or more, or 700 nm or more.

9. The hydrogel particle of claim 1, wherein the hydrogel comprises a copolymer consisting of a main monomer and a comonomer.

10. The hydrogel particle of claim 9, wherein the main monomer is selected from the group consisting of N-isopropylacrylamide, N-acryloylglycinamide, hydroxypropylcellulose, vinylcaprolactame, N-vinyl pyrrolidone, 2-hydroxyethyl methacrylate, ethylene glycol; amino acids such as aspartic acid, glutamic acid, and L-lysine; caprolactone, and vinyl methyl ether, and wherein the comonomer is selected from the group consisting of allylamine (AA), dimethylaminoethyl methacrylate (DMAEMA), dimethylaminoethyl acrylate (DMAEA), acrylic acid (AAc), polyethylene glycol (PEG), and methacrylic acid (MAAc).

11. The hydrogel particle of claim 9, wherein the hydrogel further comprises a cross-linking agent.

12. The hydrogel particle of claim 11, wherein the cross-linking agent is selected from the group consisting of N,N'-methylene-bis-diacrylamide (MBA), polyethylene glycol (PEG) PEG dihydroxyl, PEG diamine, PEG dioxyamine, PEG dichloride, PEG dibromide, PEG diazide, PEG dithiol, PEG dialdehyde, PEG diepoxide, PEG diacrylate, PEG dimethacrylate, PEG diacetic acid, PEG disuccinic acid, PEG discuccinimidyl carboxy methyl ester, poly(ε-caprolactone)diacrylate, poly(ε-caprolactone)dimethacrylate, polylactide diacrylate, polylactide dimethacrylate, poly(lactide-co-glycolide)diacrylate, poly(lactide-co-glycolide)dimethacrylate, poly(ε-caprolactone-b-ethylene glycol-b-ε-caprolactone)diacrylate, poly(ε-caprolactone-b-ethylene glycol-b-ε-caprolactone)dimethacrylate, poly(lactide-b-ethylene glycol-b-lactide)diacrylate, poly(lactide-b-ethylene glycol-b-lactide)dimethacrylate, poly[(lactide-co-glycolide)-b-ethylene glycol-b-(lactide-co-glycolide)] diacrylate, poly[(lactide-co-glycolide)-b-ethylene glycol-b-(lactide-co-glycolide)] dimethacrylate, poly(ε-caprolactone-co-lactide)-diacrylate, poly(ε-caprolactone-co-lactide)-dimethacrylate, poly(ε-caprolactone-co-glycolide)-diacrylate, poly(ε-caprolactone-co-glycolide)-dimethacrylate, poly[(caprolactone-co-lactide)-b-ethylene glycol-b-(caprolactone-co-lactide)]diacrylate, poly[(caprolactone-co-lactide)-b-ethylene glycol-b-(caprolactone-co-lactide)]dimethacrylate, poly[(caprolactone-co-glycolide)-b-ethylene glycol-b-(caprolactone-co-glycolide)]diacrylate, poly[(caprolactone-co-glycolide)-b-ethylene glycol-b-(caprolactone-co-glycolide)]dimethacrylate and a combination thereof.

13. The hydrogel particle of claim 11, wherein the hydrogel comprises a copolymer consisting of 50 to 97.9% by weight of the main monomer, 2 to 40% by weight of the comonomer, and 0.1 to 10% by weight of the cross-linking agent.

14. The hydrogel particle of claim 11, wherein the hydrogel comprises at least one selected from the group consisting of poly(N-isoprophylacrylamide-co-allylamine) (poly(NIPAM-co-AA), poly(N-isopropylacrylamide-co-2-(dimethylamino)ethyl methacrylate) (poly(NIPAM-co-DMAEMA)), poly(N-isopropylacrylamide-co-2-(dimethylamino)ethyl acrylate) (poly(NIPAM-co-DMAEA)), poly(N-isopropylacrylamide-co-acrylic acid) (poly(NIPAM-co-AAc)), poly(N-isopropylacrylamide-co-polyethylene glycol-acrylic acid) (poly(NIPAM-co-PEG-AAc)), and poly(N-isopropylacrylamide-co-methacrylic acid) (poly(NIPAM-co-MAAc)).

15. The hydrogel particle of claim 13, wherein the hydrogel comprises a copolymer prepared by using N-isopropylacrylamide as the main monomer, acrylic acid as the comonomer, and N, N'-methylene-bis-acrylamide (MBA) as the cross-linking agent.

16. A method for producing the deformable hydrogel particle according to claim 1, the method comprising steps of:
(i) preparing a hydrogel particle,
(ii) modifying a surface of the hydrogel particle so that a protein can bind to the surface of the hydrogel particle, and
(iii) adding the protein to the surface-modified hydrogel particle, thereby binding the protein to the surface of the hydrogel particle.

17. The method of claim 16, wherein the step of (i) preparing a hydrogel particle includes steps of:
mixing 50 to 97.9% by weight of a main monomer, 2 to 40% by weight of a comonomer, and 0.1 to 10% by weight of a cross-linking agent so that a sum of the three components is 100% by weight;
heating the resulting mixed solution to 55 to 80°C;
initiating a polymerization reaction with or without addition of an initiator;
obtaining an aqueous hydrogel solution produced according to the polymerization reaction; and
dialyzing the hydrogel aqueous solution with purified water for 2 weeks.

18. The method of claim 16, wherein the protein is an antibody or a recombinant protein.

19. A pharmaceutical composition for treating cancer, comprising a therapeutically effective amount of the hydrogel particle of any one of claims 1 to 15.

20. An immuno-oncology agent comprising the hydrogel particle of any one of claims 1 to 15.
